## Europäisches Patentamt
## European Patent Office
## Office européen des brevets

(19)

(11) Publication number: **0 013 797**
**B1**

(12)

# EUROPEAN PATENT SPECIFICATION

(45) Date of publication of patent specification: **01.12.82**

(21) Application number: **79301993.6**

(22) Date of filing: **25.09.79**

(51) Int. Cl.³: **C 07 C 45/29,**
**C 07 C 51/16,**
**B 01 J 23/46, B 01 J 23/96**

(54) **Oxidation of alcohols and aldehydes using a ruthenate catalyst.**

(30) Priority: **25.09.78 GB 3798278**

(43) Date of publication of application:
**06.08.80 Bulletin 80/16**

(45) Publication of the grant of the patent:
**01.12.82 Bulletin 82/48**

(84) Designated Contracting States:
**BE DE FR GB IT NL**

(56) References cited:
**US - A - 3 997 578**

(73) Proprietor: **JOHNSON, MATTHEY & Co., LIMITED**
**43 Hatton Garden**
**London EC1N 8EE (GB)**

(72) Inventor: **Griffith, William Petit**
**65 Richmond Park Road**
**London, SW14 (GB)**
Inventor: **Schroder, Martin**
**44 Waldemar Avenue**
**London, SW6 (GB)**

(74) Representative: **Arthur, Bryan Edward et al,**
**4 Dyers Buildings Holborn**
**London, EC1N 2JT (GB)**

Courier Press, Leamington Spa, England.

Oxidation of alcohols and aldehydes using a ruthenate catalyst

This invention relates to the oxidation of organic substrates and in particular provides a novel reagent for the oxidation, under mild conditions, of primary and secondary alcohols and aldehydes.

Certain organic substrates, that is to say, molecules or functional groups, may be oxidised using, as oxidising agent, either osmium tetroxide or ruthenium tetroxide and these reagents, if used in the presence of a suitable secondary oxidising agent, are catalytic in action in that the said secondary oxidising agent oxidises in situ the reduced form of the osmium or ruthenium tetroxide produced as a result of the oxidation of the substrate organic molecule or functional group, thus regenerating the osmium or ruthenium tetroxide. Examples of organic substrates which may be oxidised by osmium tetroxide or ruthenium tetroxide are primary and secondary alcohols, alkenes, alkynes and ethers.

It has been proposed in DE—A—2 642 671 to oxidise unsaturated primary and secondary alcohols to the corresponding aldehydes, ketones or acids in an aqueous alkaline solution in the presence of alkali metal ruthenate and a co-oxidation catalyst of sufficiently high potential, such as certain alkali metal (per) halates, to continually regenerate the alkali metal ruthenate throughout the oxidation reaction. The preferred co-oxidation catalyst is sodium periodate. In certain instances sodium hypochlorite may be used and such a use is described in Chem. Abs. Vol. 74 1971 35102 g.

In US—P—3 997 578 there is described a process of oxidising alcohols to fatty acids in an inert organic solvent using a ruthenium catalyst and a peracid oxidising agent. In the definition of "peracid", perchloric and periodic acids are specifically excluded. The oxidising agent is essentially acidic, only a small fractional molar proportion of base, if any, being added.

We have now found that particular selective oxidation of certain organic substrates may be carried out by oxidising the organic substrate in an aqueous alkaline persulphate solution containing a catalyst comprising the anion $(RuO_4)^{2-}$. The particular substrates are primary alcohols, secondary alcohols and unsaturated alcohols and aldehydes, the primary and unsaturated alcohols and the aldehydes being oxidised to the corresponding acid and the secondary alcohols being oxidised to the corresponding ketone.

The purpose of the oxidising agent is to regenerate the ruthenate ion $(RuO_4)^{2-}$ from a reduced form produced as a result of reaction with the organic substrate; preferably the oxidising agent is also used to generate the ruthenate ion in the first instance, so that the $(RuO_4)^{2-}$ anion is produced in situ in the reaction mixture.

According to a second aspect of the invention, a process for the manufacture of a catalyst suitable for use in the oxidation of an organic substrate as described above comprises adding ruthenium trihalide to an aqueous solution of the oxidising agent. Spectrophotometric measurements on such solutions show that all ruthenium is present as $(RuO_4)^{2-}$, no $(RuO_4)^-$ or $RuO_4$ being detected.

We have found that the preferred trihalide of ruthenium is the trichloride and the oxidising agent should be aqueous alkaline persulphate solution, for example potassium persulphate in potassium hydroxide. This oxidising agent will not oxidise organic substrates that the catalyst of the invention will cause to oxidise, which lead us to believe that the persulphate ion functions not only as a generator of $(RuO_4)^{2-}$ from ruthenium trichloride but also as a secondary oxidising agent to regenerate the $(RuO_4)^{2-}$ ion from the reduced form produced as result of the oxidation of the organic substrate.

The catalysts used in the process of the present invention catalyse the oxidation of organic substrates under mild conditions, for example at room temperature in air, with a molar ratio of substrate: $(RuO_4)^{2-}$ within the range 10:1 to 55:1 and preferably 45:1, reaction generally being complete within a few hours.

Substrates that are catalytically oxidised by the process of the invention include primary and secondary alcohols, unsaturated alcohols and aldehydes. Tertiary alcohols and unsaturated substrates such as alkenes and alkynes are substantially unaffected by the catalyst so that, for example, cinnamyl alcohol $C_6H_5CH=CHCH_2OH$ and 3-cyclohexene-1-methanol are catalytically oxidised to the corresponding carboxylic acids in high yields. We have found that other specific reactions may be carried out; for example by using a lower molar ratio of substrate:ruthenate of about 10:1 and a longer reaction time of about 24 hours, benzylamine is oxidised to benzonitrile as the main product.

The following table shows some examples of the process according to the invention. Experiments were carried out at room temperature in air using a catalytic solution containing $1.7 \times 10^{-4}$M ruthenate in potassium persulphate ($3 \times 10^{-2}$M) and potassium hydroxide ($2 \times 10^{-1}$M) at a substrate: $(RuO_4)^{2-}$ ratio generally of approximately 45:1.

## TABLE

| Substrate | Product | Reaction time (hours) | Yield[a] |
|---|---|---|---|
| 1° Alcohols | | | |
| benzyl alcohol | benzoic acid | $1\frac{1}{2}$ | 98% |
| 4-nitrobenzyl alcohol | 4-nitrobenzoic acid | 1 | 97% |
| 2° Alcohols | | | |
| benzhydrol | benzophenone | 5 | 95% |
| *cyclo*hexanol | *cyclo*hexanone | 2 | 64% |
| | adipic acid | | 7% |
| *cyclo*pentanol | *cyclo*pentanone | 2 | 71% |
| 1,2,3,4-tetrahydro-1-naphthol | $\alpha$-tetralone | 2 | 84% |
| benzoin | benzil[b] | 5 | 26% |
| | benzoic acid | | 68% |
| Aldehydes | | | |
| 4-nitrobenzaldehyde | 4-nitrobenzoic acid | $\frac{3}{4}$ | 98% |
| 3-chlorobenzaldehyde | 3-chlorobenzoic acid | 2 | 99% |
| benzaldehyde | benzoic acid | 1 | 98% |
| 2-hydroxybenzaldehyde | 2-hydroxybenzoic acid | 5 | 71% |
| 4-methoxybenzaldehyde | 4-methoxybenzoic acid | $2\frac{1}{2}$ | 96% |
| Unsaturated alcohols | | | |
| *trans*-cinnamyl alcohol | *trans*-cinnamic acid | $2\frac{1}{2}$ | 97% |
| 3-*cyclo*hexene-1-methanol | 3-*cyclo*-hexene-1-carboxylic acid | $4\frac{1}{2}$ | 68% |
| allyl alcohol | acrylic acid | $3\frac{1}{2}$ | 45% |

[a]Yields of isolated pure products
[b]Two phase reaction diethyl ether:water.

## Claims

1. A process for the oxidation of a primary alcohol, a secondary alcohol, an unsaturated alcohol or an aldehyde in which the primary alcohol, unsaturated alcohol or aldehyde is oxidised to the corresponding acid and the secondary alcohol to the corresponding ketone in an aqueous solution of an oxidising agent and a ruthenium catalyst, characterised in that the aqueous solution is an aqueous alkaline persulphate solution containing the anion $(RuO_4)^{2-}$.

2. A process as claimed in Claim 1 further characterised in that the aqueous solution contains potassium persulphate and potassium hydroxide.

3. A process as claimed in Claim 1 or 2 further characterised in that the process is carried out at room temperature in air using a molar ratio of substrate:$(RuO_4)^{2-}$ within the range 10:1 to 55:1.

4. A process as claimed in Claim 3 further characterised in that the molar ratio is 45:1.

5. A process as claimed in any preceding claim further characterised in that the anion $(RuO_4)^{2-}$ is generated *in situ* in the aqueous solution by the oxidising agent.

6. A process as claimed in Claim 5 further characterised in that the anion is generated from ruthenium trihalide.

7. A process as claimed in Claim 6 further characterised in that the trihalide is trichloride.

## Revendications

1. Procédé d'oxydation d'un alcool primaire, d'un alcool secondaire, d'un alcool insaturé ou d'un aldéhyde dans lequel l'alcool primaire, l'alcool insaturé ou l'aldéhyde est oxydé en l'acide correspondant et l'alcool secondaire en la cétone correspondante, dans une solution aqueuse d'un agent d'oxydation et un catalyseur à base de ruthénium, ce procédé étant caractérisé en ce que la solution aqueuse est une solution aqueuse de persulfate alcalin contenant l'anion $(RuO_4)^{2-}$.

2. Procédé selon la revendication 1, caractérisé en outre en ce que la solution aqueuse contient du persulfate de potassium et de l'hydroxyde de potassium.

3. Procédé selon la revendication 1 ou 2, caractérisé en outre en ce que le procédé est mis en oeuvre à température ambiante dans un courant d'air, le rapport molaire substrat/$(RuO_4)^{2-}$ étant compris entre 10/1 et 55/1.

4. Procédé selon la revendication 3, caractérisé en outre en ce que le rapport molaire précité est de l'ordre de 45/1.

5. Procédé selon l'une quelconque des revendications précédentes, caractérisé en outre en ce que l'anion $(RuO_4)^{2-}$ est obtenu *in situ* dans la solution aqueuse par l'agent d'oxydation.

6. Procédé selon la revendication 5, caractérisé en outre en ce que l'anion est obtenu à partir de trihalogénure de ruthénium.

7. Procédé selon la revendication 6, caractérisé en outre en ce que le trihalogénure est le trichlorure.

## Patentansprüche

1. Verfahren zum Oxidieren eines Primäralkoholes, eines Sekundäralkoholes, eines ungesättigten Alkoholes oder eines Aldehyds in einer wässrigen Lösung aus einem oxidierenden Agens und einem Rutheniumkatalysator, wobei der Primäralkohol, der ungesättigte Alkohol oder das Aldehyd zu der entsprechenden Säure und der Sekundäralkohol zum entsprechenden Keton oxidiert, dadurch gekennzeichnet, daß die wässrige Lösung eine wässrige Alkalinpersulfatlösung mit dem Anion $(RuO_4)^{2-}$ ist.

2. Verfahren nach Anspruch 1, dadurch gekennzeichnet, daß die wässrige Lösung Kaliumpersulfat und Kaliumhydroxid enthält.

3. Verfahren nach Anspruch 1 oder 2, dadurch gekennzeichnet, daß es in Luft bei Raumtemperatur unter Verwendung eines Molverhältnisses des Substrates $(RuO_4)^{2-}$ im Bereich 10:1 bis 55:1 durchgeführt wird.

4. Verfahren nach Anspruch 3, gekennzeichnet durch ein Molverhältnis 45:1.

5. Verfahren nach einem der Ansprüche 1 bis 4, dadurch gekennzeichnet, daß das Anion $(RuO_4)^{2-}$ in situ in der wässrigen Lösung durch das oxidierende Agens erzeugt wird.

6. Verfahren nach Anspruch 5, dadurch gekennzeichnet, daß das Anion aus Rutheniumtrihalid erzeugt wird.

7. Verfahren nach Anspruch 6, dadurch gekennzeichnet, daß das Trihalid Trichlorid ist.